# EUROPEAN PATENT SPECIFICATION

(11) **EP 1 836 906 B1**
(45) Date of publication and mention of the grant of the patent: **01.07.2009**
(21) Application number: 07103303.9
(22) Date of filing: 01.03.2007
(51) Int. Cl.: A23L 1/305, A61K 38/08, A61K 38/04, A61P 3/06

(54) **Healthy food product**
Gesundes Nahrungsmittel
Produit alimentaire sain

(30) Priority: 24.03.2006 EP 06075695; 24.03.2006 EP 06075696
(43) Date of publication of application: 26.09.2007
(73) Proprietor: Unilever N.V., 3013 AL Rotterdam (NL); Unilever PLC, London EC4Y 0DY (GB)
(72) Inventor: Lin, Yuguang, Unilever R&D Vlaardingen B.V., 3133 AT Vlaardingen (NL); Vermeer, Mario Adriaan, Unilever R&D Vlaardingen B.V., 3133 AT Vlaardingen (NL)
(74) Representative: Joppe, Hermina Laura Petronella

(56) References cited:
- WO-A-01/24789
- US-A1- 2001 005 714
- US-A1- 2002 147 144
- US-A1- 2003 191 057
- PATENT ABSTRACTS OF JAPAN vol. 2003, no. 12, 5 December 2003 (2003-12-05) & JP 2004 099447 A (KYOWA HAKKO KOGYO CO LTD), 2 April 2004 (2004-04-02)
- LING W H ET AL: "MINIREVIEW DIETARY PHYTOSTEROLS: A REVIEW OF METABOLISM, BENEFITS AND SIDE EFFECTS" LIFE SCIENCES, PERGAMON PRESS, OXFORD, GB, vol. 57, no. 3, 1995, pages 195-206, XP000995149 ISSN: 0024-3205
- NAGAOKA S ET AL: "Identification of novel hypocholesterolemic peptides derived from bovine milk beta -lactoglobulin" BIOCHEMICAL AND BIOPHYSICAL RESEARCH COMMUNICATIONS, ACADEMIC PRESS INC. ORLANDO, FL, US, vol. 281, 2001, pages 11-17, XP002256619 ISSN: 0006-291X

## Description

### Field of the Invention

The present invention concerns a food product comprising an ingredient which has a blood cholesterol lowering effect, preferably in amounts sufficient to obtain a blood cholesterol lowering effect if the food product is used according to the common needs of the consumer.

### Background Art

The variation in the mean plasma total cholesterol concentration among populations is highly correlated with the variation in the extent of atherosclerosis and in the incidence of Coronary Heart Disease (CHD), which is one of the major causes of death in the Western society. Populations with a low cholesterol level (less than 180 mg/dl (4.7 mmol/L) are found to be less sensitive to atherosclerosis and coronary heart disease, whereas those with mean cholesterol levels above 220 mg/dl (5.7 mmol/L; hypercholesterolaemia) have increased rates of death due to CHD. Thus, there is a clear need for a method by which the cholesterol level can be lowered.

Sterols and sterol esters have been proposed as blood cholesterol lowering ingredients, for example in European patent application no. EP 619 952 (Amano), a food additive is described, by which the level of cholesterol in food is lowered by treating the food with the food additive. Upon using the additive, (y-oryzanol being mentioned), a complex with the cholesterol in the food is formed. The food containing the additive can therefor not be applied to lower the blood cholesterol level in mammals.

In WO 92/19640 (Raision Margariini Oy) a substance of β-sitostanol fatty acid ester is described that can be used as such or added to food. It is described that an ordinary diet contains plant sterols 100-300 mg/day, and that these sterols are poorly absorbed from the intestines. In WO 92/19640, it is thus argued that the use of the plant sterols cannot be used for reducing the serum cholesterol levels in the human diet. The cholesterol lowering effect of plant sterol or stanol or their esters has been described to be mainly via inhibiting cholesterol incorporation into micelles.

EP 828,434 describes that a lowering of blood cholesterol level is obtainable by the regular consumption of fat based food products which comprise at least one compound of the group consisting of phytosterol and oryzanol, and mixtures thereof.

Other ingredients have also been suggested for the lowering of blood cholesterol. For example WO 01/24789 describes β-peptides as active substances for the reduction of cholesterol-uptake and lipid-uptake from the gut. In order to act as a precipitant these peptides need at least two positive charges. However the use of β-peptides sometimes is not desired because they have a different chemical structure to naturally occurring peptides.

US 2001/0005714 describes natural or variant apo-proteins and other proteins and peptides having an amphipathic α-helix composed of at least 15 amino acids. These molecules inhibit the uptake of cholesterol and other lipids from the gut.

JP 2004099447 describes a specific peptide with amino acid sequence Val-Ala-Trp-Trp-Met-Tyr, which occurs in soy protein, can be used in food products to have cholesterol-lowering effect.
EP 1,046,396 describes a composition containing soy protein and plant sterols having synergistic cholesterol lowering effect. However, factions (peptides) responsible for the cholesterol lowering effect of soy protein have not been described.

WO 01/37681 describes a composition containing plant sterols and an isolated water-soluble protein, such as soy protein or caseinate, and optionally also an emulsifier. The composition has no extra cholesterol lowering effect compared to plant sterols alone. US 6113972 describes a complex of plant sterols with protein, where the complex increases the bio-availability of the plant sterols. No cholesterol lowering effect is attributed to the protein.

EP 790,060 discloses a soy protein-hydrolysate-phospholipid complex for lowering plasma cholesterol.

WO 03/055324 A1 describes compositions containing plant sterols and hydrolysates of protein products, prepared by using aproteolytic enzyme preparation, to lower plasma cholesterol.

FDA has approved a health claim about the role of soy protein in reducing the risk of cardiovascular disease by lowering plasma cholesterol. In order to qualify for this health claim, a food must contain at least 6.25 grams of soy protein per serving, the amount being one-fourth of the effective level of 25 grams per day.

There still exists a need for alternative food products comprising an ingredient which has a blood cholesterol lowering effect. In particular there is a need for food products comprising ingredients that may inhibit cholesterol absorption via a defined mechanism, for example by interrupting cholesterol micelle formation.

### Summary of the Invention

It is an object of the present invention to provide a food product comprising peptides which are able to inhibit the uptake of cholesterol via interfering in micelle formation. It has been found that suitable peptides for use in these food products preferably satisfy a number of criteria. It is believed that if these peptides are used in food products in specific amounts, they contribute to the control of blood cholesterol levels. Although applicants by no means wish to be bound by any theory, it is believed that the peptides satisfying the criteria indicated below disrupt the formation of cholesterol micelles in the gut, therewith preventing or reducing the uptake of cholesterol through the intestinal wall.

Accordingly in a first aspect the invention relates to a food product comprising a peptide, said peptide being characterized by having a number of amino acids from 6 to 20, and wherein the peptide is substantially water-insoluble at 20°C and a pH of 6.5, as evidenced by a measurable turbid appearance if 10 mg of the peptide is mixed with 10 ml of de-mineralised water at 20°C and pH 6.5, and wherein the peptide is substantially composed of neutral polar amino acids selected from the group of G, S, T, Y, C, Q and N.

Preferably said peptide has a molecular weight between 450 and 3,000 Dalton. Preferably said peptides are composed of neutral polar amino acids, excluding negatively charged amino acids. Also preferably the water-insoluble peptides for use in food products of the invention are not β-peptides, but rather are peptides containing amino acids commonly present in naturally occurring peptides, e.g. α-peptides.

Preferably the food product is selected from the group consisting of drinks, dairy type products, frozen confectionery products or spreads or margarines. Also preferable the amount of the peptides as defined above in the food product is art least 0.1 g/kg, preferably from 1 to 200 g/kg, most preferably from 2 to 30 g/kg.

The invention also relates to the use of these food products in the preparation of a nutritional product suitable for lowering blood cholesterol levels.

### Detailed Description of the Invention

Peptides for use in food products according to the invention are water-insoluble in water at 20°C and a pH of 6.5. This can for example be evidenced by mixing 10 mg of the peptide into 10 ml of de-mineralised water at 20°C and pH 6.5, and stirring. A (measurable) turbid appearance of the solution after stirring is indicative of the fact that the peptide is water-insoluble.

The peptides for use in food products of the invention are characterized by having a number of amino acids from 6 to 20, and by being composed of neutral polar amino acids selected from the group of G, S, T, Y, C, Q and N, provided the resulting peptide is substantially water-insoluble.

For the purpose of this invention the following naturally occurring amino acids are classified as hydrophobic amino acids: Alanine (A), Valine (V), Leucine (L), Isoleucine (I), Proline (P), Tryptophan (W), Phenylalanine (F), Methionine (M).

For the purpose of this invention the following naturally occurring amino acids are classified as neutral polar amino acids: Glycine (G), Serine (S), Threonine (T), Tyrosine (Y), Cysteine (C), Glutamine (Q) and Asparagine (N).

The number of amino acids in the peptide is from 6 to 12, more preferably from 6 to 10 and most preferably from 6 to 8. Preferably the net charge of the peptide is 0 or positively charged.

The net charge of a peptide can be determined by summation of the charges of the individual amino acids of the peptide at pH 6.5.

Amino acids having a charge a +1 at pH 6.5 are Lysine (K), Arginine (R) and Histidine (H). Amino acids having a charge of -1 at pH 6.5 are Aspartic acid (D) and Glutamic acid (E).

The remaining natural amino acids have a charge of 0 at pH 6.5 and are Alanine (A), Valine (V), Leucine (L), Isoleucine (I), Proline (P), Tryptophan (W), Phenylalanine (F), Methionine (M), Glycine (G), Serine (S), Threonine (T), Tyrosine (Y), Cysteine (C), Glutamine (Q) and Asparagine (N).

Preferably the majority of the amino acids of the peptide is composed of one kind of neutral polar amino acid selected from the group of G, S, T, Y, C, Q and N. For the purpose of this invention the term 'the majority of' means that at least half or more of the total number of amino acids of the peptide (e.g. for a hexa-peptide that is at least 3 amino acids, for a heptapeptide that is at least 4 amino acids).

Even more preferably the amino acids of the peptide are composed of only one kind of amino acid selected from the group of G, S, T, Y, C, Q and N (e.g. GGGGGGGG, YYYYYY)

Specific examples of suitable peptides are provided in Tables 1 and 3.

| **Table 1, Examples of water-insoluble peptides for use in food products according to the invention** | |
|---|---|
| **Peptide** | **Amino acid sequence** |
| I | GGWWMY* |
| II | YYYYYY |
| III | YYYYYYYY |

| | |
|---|---|
| * not according to the invention | |

The peptides may be obtained or manufactured by any method known in the art, including synthesis of the pure peptides.

Food products according to the invention preferably are selected from the group consisting of drinks, dairy type products, frozen confectionery products or spreads/margarine. The amount of the peptides in the food product is preferably at least 0.1 g/kg, more preferred from 1 to 200 g/kg , most preferred from 2 to 30 g/kg. These preferred types of food products are described in some detail below.

Preferably the amount of peptides in these food products is chosen such that per serving an amount of 0.1 to 10 gram peptides is present. Suitable serving sizes are indicated in Table 2.

| **Table 2, Suitable serving sizes** | |
|---|---|
| **Food product** | **Serving size** |
| Fruit juice | 200 gram |
| Milk type drink | 125 gram |
| Yoghurt | 125 gram |
| Frozen confectionery product | 75 gram |
| Spread | 15 gram |
| Sauce | 40 gram |
| Bar | 60 gram |

### Fruit juice products

Examples of fruit juice products according to the invention are juices derived from citrus fruit like orange and grapefruit, tropical fruits, banana, peach, peer, strawberry, to which the peptides and optionally one or more further heart health ingredients are added.

### Dairy type products

Examples of dairy products according to the invention are milk, dairy spreads, cream cheese, milk type drinks and yogurt, to which the peptides and optionally one or more heart health ingredients are added.
The food product may be used as such as a milk type drink. Alternatively flavor or other additives may be added. A dairy type product may also be made by adding the peptides to water or to a dairy product.

An example of a composition for a yoghurt type product is about 50-80 wt% water, 1-10 wt% of the peptides and optionally one or more heart health ingredients, 0-15 wt% whey powder, 0-15 wt% sugar (e.g. sucrose), 0.01-1 wt% yoghurt culture, 0-20 wt% fruit, 0.05-5 wt% vitamins and minerals, 0-2 wt% flavour, 0-5 wt% stabiliser (thickener or gelling agent). To the yoghurt, fruit may be added.

A typical serving size for a yoghurt type product could be from 50 to 250 g, generally from 80 to 200 g.

### Frozen Confectionery Products

For the purpose of the invention the term frozen confectionery product includes milk containing frozen confections such as ice-cream, frozen yoghurt, sherbet, sorbet, ice milk and frozen custard, water-ices, granitas and frozen fruit purees.

Preferably the level of solids in the frozen confection (e.g. sugar, fat, flavouring etc) is more than 3 wt%, more preferred from 10 to 70 wt%, for example 40 to 70 wt%.

Ice cream will typically comprise 0 to 20 wt% of fat, 1 to 10 wt% of the peptides and optionally one or more heart health ingredients, sweeteners, 0 to 10 wt% of non-fat milk components and optional components such as emulsifiers, stabilisers, preservatives, flavouring ingredients, vitamins, minerals, etc, the balance being water. Typically ice cream will be aerated e.g. to an overrun of 20 to 400 %, more specific 40 to 200 % and frozen to a temperature of from -2 to -200 °C, more specific -10 to -30 °C. Ice cream normally comprises calcium at a level of about 0.1 wt%.

### Oil and water containing emulsions

Advantageously the food product is an oil and water containing emulsion, for instance a spread or a margarine. Oil and water emulsion is herein defined as an emulsion comprising oil and water and includes oil in water (O/W) emulsions and water in oil emulsions (W/O) and more complex emulsions for instance water-in-oil-in-water (W/O/W/O/W) emulsions. Oil is herein defined as including fat.

Preferably a spread according to the invention comprises 30-90 wt% vegetable oil, 1-5 wt% of the peptides, and optionally one or more further heart health ingredients in suitable amounts. Advantageously a spread has a pH of 4.2-6.0.

An example of a composition of a spread is 37.45 wt% of a fat blend, 52.8 wt% water, 0.15 wt% lecithin, 0.2 wt% monoglyceride, 0.1 wt% flavour, 0.5 wt% sodium chloride, 0.1 wt% potassium sorbate, 0.1 wt% sweet buttermilk powder, 6 wt% starch, 2.5 wt% of the peptides.

### Other food products

Other food products according to the invention can be prepared by the skilled person based on common general knowledge, the peptides and optionally one or more heart health ingredients in suitable amounts. Examples of such food products are baked goods, snacks, sauces, bars etc.

Food products of the invention preferably contain further heart health ingredients. Particularly preferred is the use of sterols, such as for example phytosterols or phytostanols, in food products of the invention. Preferred sterols for use in the food products of the invention are described in more detail below. For the purpose of the invention the term sterols refer to sterols, stanols, their analogues and their esters.

### Phytosterols, Phytostanols, Analogues and Derivatives

Typically, the phytosterols, phytostanols and their analogues and derivatives may be selected from one or more of phytosterols, phytostanols, synthetic analogues of phytosterols and phytostanols and esterified derivatives of any of the foregoing, and mixtures of any of these. The total amount of such substances in a food product is preferably from 0.01% to 20%, more preferably from 0.1% to 15%, still more preferably from 0.2% to 8%, and most preferably from 0.3% to 8% by weight of the food product composition.

Preferably, the intake per serving of such sterol-type component of the combination is from 0.1g to 3g, more preferably from 1.5g to 2.5g, especially from 2g to 2.25g per serving.

Phytosterols, also known as plant sterols or vegetable sterols can be classified in three groups, 4-desmethylsterols, 4-monomethylsterols and 4,4'-dimethylsterols. In oils they mainly exists as free sterols and sterol esters of fatty acids although sterol glucosides and acylated sterol glucosides are also present. There are three major phytosterols namely beta-sitosterol, stigmasterol and campesterol.
The phytostanols are the respective 5α- saturated derivatives of phytosterols such as sitostanol, campestanol and their derivatives.

Synthetic analogues of any of the phytosterols or phytostanols (which include chemically modified natural phytosterols or phytostanols) may be used.

Preferably the phytosterol or phytostanol is selected from the group comprising fatty acid ester of β-sitosterol, β-sitostanol, campesterol, campestanol, stigmasterol, stigmastanol and mixtures thereof.

The optional phytosterol or phytostanol materials recited above may optionally be provided in the form of one or more fatty acid esters thereof. Mixtures of esterified and non-esterified materials may also be used.

Thus, any of the sterols e.g. phytosterols or phytostanols and their synthetic analogues used in the present invention are preferably esterified with a fatty acid. Preferably, they are esterified with one or more C₂₋₂₂ fatty acids. For the purpose of the invention the term C₂₋₂₂ fatty acid refers to any molecule comprising a C₂₋₂₂ main chain and at least one acid group. Although not preferred within the present context the C₂₋₂₂ main chain may contain 1-6 double bonds, be partially substituted or side chains may be present. Preferably, however the C₂₋₂₂ fatty acids are linear molecules comprising one or two acid group(s) as end group(s). Most preferred are linear C₈₋₂₂ fatty acids as occur in natural liquid oils.

Preferably food products of the invention comprise a combination of the peptides as described above and sterols, wherein the weight ratio of peptides to sterols is from 100 : 1 to 1 : 50, more preferred 50 : 1 to 1 : 30, most preferred from 5 : 1 to 1 : 15.

The invention will be further illustrated in the following examples.

### Example I

### Method of preparing the peptides

### Synthetic peptides

Peptides according to the invention may be made by any synthesizing method known in the art.

Evaluation of the effect of peptides on cholesterol solubility in mixed micelles.

Cholesterol is a hydrophobic compound, which is water-insoluble and forms crystals in an aqueous environment. This property makes it difficult for cholesterol to move from the lumen towards the brush border membrane in the intestine, where cholesterol is taken up by enterocytes. In the intestinal lumen amphiphilic molecules, such as monoacylglycerols (MAG), free fatty acids (FFA), and lysophospholipids (LPL) are generated via enzymatic hydrolysis of foods. Bile acids, phospholipids (PL) and free cholesterol are secreted into the duodenum from the gallbladder. These compounds (bile acids, FFA, MAG, PL, LPL and cholesterol) can form a structure called dietary mixed micelles. Dietary mixed micelles can dissolve hydrophobic cholesterol to prevent it from crystallization in an aqueous environment.

Therefore, dietary mixed cholesterol micelles play an important role in cholesterol absorption, via acting as vehicles that transport cholesterol towards the intestinal wall.

To form mixed micelles *in vitro,* 0.1 mM lecithin, 0.05 mM monoolein, 0.1 mM oleic acid and 80 µM cholesterol are mixed in a test tube and dried under a stream of N₂. After drying, a 2 mM bile acid mix (BA-mix) in PBS pH 6.5 is added to this lipid mixture. Fluorescent labelled cholesterol (NBD-cholesterol) is used to monitor the incorporation of cholesterol into the mixed micelles.

For testing, peptides (1 mg/ml) as indicated in Table 3 are added to the BA-mix and then mixed with lipid-mixtures as described. The whole mixture is vortexed for 15 seconds, followed by an ultrasonic treatment in a water bath for 30 minutes. From this mixture a sample is taken to measure total NBD-cholesterol concentration. The remaining micelle solution is centrifuged in a table-top ultracentrifuge at 50.000g for 30 minutes at room temperature.

After centrifugation, samples from the supernatant are taken for the determination of solubilised NBD-cholesterol, which indicates the amount of cholesterol incorporated into mixed micelles. The pellet containing crystallised cholesterol is discarded. NBD-cholesterol is measured at 375/465 nm using a fluorescence plate reader.

Mixed micelle mixtures containing no peptides are used as control (100%). For each example the percentage of incorporated Cholesterol is calculated by dividing the amount of incorporated cholesterol for the tested composition by the amount of incorporated cholesterol in the control sample.

Table 3 provides for various peptides the % cholesterol incorporation into mixed micelles. This table also indicates which peptides are substantially water-insoluble at 20 C and pH 6.5.

**Table 3. The inhibitory effect of water-insoluble peptides on the incorporation of cholesterol into mixed micelles**

| Peptide category | Amino acid sequence | Cholesterol incorporation |
|---|---|---|
| | | % of control |
| No peptides (control) | --- | 100 |
| Insoluble peptides | GGGGGG | 34 |
| | GGGGGGGG | 32 |
| | YYYYYY | 40 |
| Soluble peptides | VEWEME* | 87 |
| | EAEWEY* | 86 |
| | GSGGSG | 90 |
| | DDDDDD* | 155 |

| | | |
|---|---|---|
| * not according to the invention | | |

Conclusion: It was found that water-insoluble peptides, preferably composed of neutral polar amino acids are capable of in inhibiting the incorporation of cholesterol into mixed micelles.

### Example II

Food products according to the invention.

Example IIIa: 0.3 g of hexa-peptide YYYYYY can be mixed into a commercially available 125 grams serving of fruit yoghurt.

Example IIIb: 0.5 g of octa-peptide GGGGGGGG and 3 g of a sterol ester (beta-sitosterol esterified with sunflower oil) can be added to a commercially available serving of 200 grams drink-yogurt.

## Claims

1. Food product comprising a peptide, said peptide being **characterized by** having a number of amino acids from 6 to 20, and wherein the peptide is substantially water-insoluble at 20°C and a pH of 6.5, as evidenced by a measurable turbid appearance if 10 mg of the peptide is mixed with 10 ml of de-mineralised water at 20°C and pH 6.5, and wherein the peptide is composed of neutral polar amino acids selected from the group of G, S, T, Y, C, Q and N.

2. Food product according to claim 1, **characterized in that** said peptide has a number of amino acids from 6 to 12, more preferably from 6 to 10 and most preferably from 6 to 8.

3. Food product according to claim 1 or 2, **characterized in that**, said peptide has a molecular weight of from 450 to 3,000 Dalton.

4. Food product according to any one of claim 1 to 3, **characterized in that**, the net charge of said peptide is 0.

5. Food product according to any one of claim 1 to 4, **characterized in that**, said peptide is composed of one kind of neutral polar amino acid selected from the group of G, S, T, Y, C, Q and N.

6. Food product according to any one of claim 1 to 5, selected from the group consisting of drinks, dairy type products, frozen confectionery products and spreads or margarines.

7. Food product according to any of claim 1 to 6, further comprising sterols in an amount of from 0.01% to 20%, more preferably from 0.1% to 15%, still more preferably from 0.2% to 8%, and most preferably from 0.3% to 8% by weight.

8. Food product according to claim 7, wherein the weight ratio of said peptides to sterols is from 100 : 1 to 1 : 50, more preferred 50 : 1 to 1 : 30, most preferred from 5 : 1 to 1 : 15.

9. Food product according to any one of claim 1 to 8, **characterized in that**, the amount of said peptides in the food product is at least 0.1 g/kg, more preferably from 1 to 200 g/kg and most preferably from 2 to 30 g/kg.

10. Use of food product according to any one of claim 1 to 9 in the preparation of a nutritional product suitable for lowering blood cholesterol levels.

## Patentansprüche

1. Nahrungsmittelprodukt, das ein Peptid umfasst, wobei das Peptid **dadurch gekennzeichnet ist, dass** es eine Anzahl von Aminosäuren von 6 bis 20 hat und wobei das Peptid bei 20 °C und einem pH von 6,5 im Wesentlichen wasserunlöslich ist, wie es durch ein messbares trübes Aussehen bewiesen wird, wenn 10 mg des Peptids mit 10 ml entmineralisiertem Wasser bei 20 °C und einem pH von 6,5 gemischt werden, und wobei das Peptid aus neutralen polaren Aminosäuren gebildet ist, die aus der Gruppe aus G, S, T, Y, C, Q und N ausgewählt sind.

2. Nahrungsmittelprodukt gemäß Anspruch 1, **dadurch gekennzeichnet, dass** das Peptid eine Anzahl von Aminosäuren von 6 bis 12, bevorzugter von 6 bis 10 und am bevorzugtesten von 6 bis 8 hat.

3. Nahrungsmittelprodukt gemäß Anspruch 1 oder 2, **dadurch gekennzeichnet, dass** das Peptid ein Molekulargewicht von 450 bis 3000 Dalton hat.

4. Nahrungsmittelprodukt gemäß einem der Ansprüche 1 bis 3, **dadurch gekennzeichnet, dass** die Nettoladung des Peptids 0 ist.

5. Nahrungsmittelprodukt gemäß einem der Ansprüche 1 bis 4, **dadurch gekennzeichnet, dass** das Peptid aus einer Art neutraler polarer Aminosäure, ausgewählt aus der Gruppe von G, S, T, Y, C, Q und N, gebildet ist.

6. Nahrungsmittelprodukt gemäß einem der Ansprüche 1 bis 5, ausgewählt aus der Gruppe, bestehend aus Getränken, Produkten des Milchtyps, gefrorenen Konfektprodukten und Aufstrichen oder Margarinen.

7. Nahrungsmittelprodukt gemäß einem der Ansprüche 1 bis 6, das außerdem Sterole in einer Menge von 0,01 Gewichts-% bis 20 Gewichts-%, bevorzugter von 0,1 Gewichts-% bis 15 Gewichts-%, noch bevorzugter von 0,2 Gewichts-% bis 8 Gewichts-% und am bevorzugtesten von 0,3 Gewichts-% bis 8 Gewichts-% umfasst.

8. Nahrungsmittelprodukt gemäß Anspruch 7, wobei das Gewichtsverhältnis der Peptide zu Sterolen 100 : 1 bis 1 : 50, bevorzugter 50 : 1 bis 1 : 30, am bevorzugtesten von 5 : 1 bis 1 : 15 ist.

9. Nahrungsmittelprodukt gemäß einem der Ansprüche 1 bis 8, **dadurch gekennzeichnet, dass** die Menge der Peptide in dem Nahrungsmittelprodukt wenigstens 0,1 g/kg, bevorzugter 1 bis 200 g/kg und am bevorzugtesten 2 bis 30 g/kg ist.

10. Verwendung eines Nahrungsmittelprodukts gemäß einem der Ansprüche 1 bis 9 bei der Herstellung eines nutritionalen Produkts, das zur Senkung der Blutcholesterinspiegel geeignet ist.

## Revendications

1. Produit alimentaire comprenant un peptide, ledit peptide étant **caractérisé en ce qu'**il présente un nombre d'acides aminés de 6 à 20, et le peptide étant substantiellement insoluble dans l'eau à 20°C et à un pH de 6,5, mis en évidence par un aspect trouble mesurable si 10 mg de peptide sont mélangés à 10 ml d'eau déminéralisée à 20°C et à un pH de 6,5, le peptide étant constitué d'acides aminés polaires neutres choisis dans le groupe de G, S, T, Y, C, Q et N.

2. Produit alimentaire selon la revendication 1, **caractérisé en ce que** ledit peptide a un nombre d'acides aminés de 6 à 12, de manière davantage préférée, de 6 à 10 et de manière préférée entre toutes, de 6 à 8.

3. Produit alimentaire selon la revendication 1 ou 2, **caractérisé en ce que** ledit peptide a un poids moléculaire de 450 à 3 000 daltons.

4. Produit alimentaire selon l'une quelconque des revendications 1 à 3, **caractérisé en ce que** la charge nette dudit peptide est de 0.

5. Produit alimentaire selon l'une quelconque des revendications 1 à 4, **caractérisé en ce que** ledit peptide est constitué d'une sorte d'acide aminé polaire neutre choisi dans le groupe de G, S, T, Y, C, Q et N.

6. Produit alimentaire selon l'une quelconque des revendications 1 à 5, choisi dans le groupe constitué par les boissons, les produits de type laitier, les produits de confiserie congelés et les pâtes à tartiner ou margarines.

7. Produit alimentaire selon l'une quelconque des revendications 1 à 6, comprenant en outre des stérols en une quantité de 0,01 % à 20 %, de manière davantage préférée, de 0,1 % à 15 %, de manière encore davantage préférée, de 0,2 % à 8 % et de manière préférée entre toutes, de 0,3 % à 8 % en poids.

8. Produit alimentaire selon la revendication 7, dans lequel le rapport en poids desdits peptides aux stérols est de 100:1 à 1:50, de manière davantage préférée, de 50:1 à 1:30, de manière préférée entre toutes, de 5:1 à 1:15.

9. Produit alimentaire selon l'une quelconque des revendications 1 à 8, **caractérisé en ce que** la quantité desdits peptides dans le produit alimentaire est d'au moins 0,1 g/kg, de manière davantage préférée de 1 à 200 g/kg et de manière préférée entre toutes, de 2 à 30 g/kg.

10. Utilisation d'un produit alimentaire selon l'une quelconque des revendications 1 à 9, dans la préparation d'un produit nutritionnel approprié pour réduire les taux de cholestérol sanguin.
